# EUROPEAN PATENT APPLICATION

(11) **EP 2 110 148 A1**
(43) Date of publication of application: **21.10.2009**
(21) Application number: 08007642.5
(22) Date of filing: 18.04.2008
(51) Int. Cl.: A61L 31/16

(54) **Polymer-free stents comprising an endothelin receptor antagonist**

(71) Applicant: von Korn, Hubertus, 67435 Geinsheim/Neustadt (DE); Lauer, Bernward, 99425 Weimar (DE)
(72) Inventor: von Korn, Hubertus, 67435 Geinsheim/Neustadt (DE); Lauer, Bernward, 99425 Weimar (DE)
(74) Representative: Ricker, Mathias

(57) **Abstract**

Stent, **characterized in that** it is a bare metal stent, which comprises an endothelin receptor antagonist. Said stent is drug-eluting and is for use as a medicament, specifically for the treatment of stenosis or for the treatment of coronary artery diseases.

## Description

The present invention pertains to a stent, i.e. a polymer-free stent comprising an endothelin receptor antagonist. Said stent is drug-eluting and is for use as a medicament, specifically for the treatment and/or for the prevention of stenosis, coronary artery diseases and peripheral arterial disease. In a preferred embodiment the polymer-free stent comprises tezosentan as an endothelin receptor antagonist.

Coronary artery disease (CAD) is a widespread disease. Alone in the US, about 14,000,000 men and women are affected. In many patients with CAD, percutaneous transluminal coronary angioplasty (PTCA) is a widely used treatment method. The employment of PTCA has grown rapidly in the late 70ies. However, stenosis following PTCA remains to be a significant problem. From 25 % to 35 % of the patients develop restenosis within 1 to 3 months. Restenosis results in significant more morbidity and mortality and frequently necessitates further interventions such as repeated angioplasty or coronary by-pass surgery.

Peripheral arterial disease (PAD) is narrowing of one or more arteries (blood vessels), which can result from atherosclerosis, inflammatory processes leading to stenosis. It mainly affects arteries that take blood to the legs. The condition is also known as Peripheral vascular disease (PVD). The narrowing of the arteries is caused by atheroma. Atheroma is like fatty patches or "plaques" that develop within the inside lining of the arteries. Atheroma can develop in any artery, but the common arteries affected are the arteries taking blood to the heart, arteries taking blood to the brain and arteries taking blood to the legs. The prevalence of PAD in people aged over 55 years is 10-25% and increases with age; 70-80% of affected individuals are asymptomatic.

The processes responsible for stenosis after PTCA are not completely understood. However, direct and indirect evidence supports the concept of intimal hyperplasia or proliferation of smooth muscle cells of medial or possibly of intimal origin as the fundamental process. The histological status of the coronary arteries in patients who have undergone PTCA within the previous six months and developed recurrent lesions has already been described. The restenotic lesions in all cases consisted of intimal hyperplasia. In addition, intimal hyperplasia or thickening was consistently observed in all patients after PTCA whether or not restenosis occurred. Direct evidence has come from the study of restenotic lesions in coronary and peripheral arteriosclerotic arteries treated with arterectomy. Tissue specimen from these lesions consisted almost entirely of hyperplastic smooth muscle. Medial smooth muscle cells are the major component of the arterial wall, and the only major reparative or reactive response of the arterial wall to various types of injury, either mechanical or inflammatory, is intimal proliferation from these cells *(*Liu et al., Circulation 79: 1374-1387 (1989*)).*

It is already known to use drug eluting stents (DES) in reducing the incidence of restenosis. Stents are medical implants mostly made of metal, e.g. in the form of a wire netting or a wire mesh. They usually have a very smooth surface due to electropolishing treatment. Onto this surface, a polymeric coating is applied. This polymeric layer serves as a carrier of an active pharmaceutical ingredient. During application, this active pharmaceutical ingredient is released or eluted from this polymeric layer. Exemplary stents are known in the art as Taxus Stent or Cypher Stent. The Cypher Stent (e.g. Cordis Medizinische Apparate GmbH, a Johnson&Johnson company, Langenfeld, Germany) elutes sirolimus (rapamycin), a macrocyclic immunosuppressive drug. The Taxus Stent (Boston Scientific) elutes paclitaxel, a cytostatic drug.

A process for the manufacture of a stent is e.g. known from DE 10235868 A1.

Stents are applied in the therapeutical expansion of the coronary blood vessels or for the prevention of restenosis. The implanted stent serves as a support for the blood vessel. The implant stabilizes the wall of the blood vessel mechanically, and the drug which is released or eluted from the polymer layer, stabilizes the wall therapeutically.

Such polymer-coated drug eluting stents are known to the person skilled in the art from, e.g. WO 01/70295, US 2005/0175667 A1. WO 01/70295 relates to poly-LD-lactic acid coated stents with endothelin-1 receptor A antagonist.

Said stents have reduced the need for repeated revascularization procedures. Accordingly, said medical devices are now used in almost 90 % of the stent implantation procedures performed in US hospitals. However, the increasing number of patients receiving DESs and the availability of long-term follow-up data have brought up some concern regarding the safety of these medical devices or implants. At the core of these concerns is the potential of increased inflammatory and thrombogenic responses and life-threatening consequences. These adverse effects have *inter alia* been attributed to the polymers employed for the delivery of the active pharmaceutical ingredients or drugs. After elution of the drug, the stent and therewith the polymer coated thereon, remain in the (coronary) artery. However, polymers are known to induce chronic inflammatory reaction.

The Cypher- and Taxus Stent have been shown in randomized trials to effectively inhibit the process of neointimal proliferation resulting in restenosis reduction (Morice et al., N Engl Med 2002; 346; 1773-1780), but restenosis or late stent thrombosis are not completely eliminated. Delayed and incomplete re-endothelialization may also occur (J. Pollak, J Kardiol 2007; 14 (1-2), 31- 34).

It has also already been suggested to employ drug-coated stents, which are polymer-free. Such stents are based on bare metal. These bare metal-based stents have a microscopically rough, porous surface made up of micropores with a density of approximately 1,000,000 pores per cm² and an average micropore depth of approximately 2 micrometers that permits absorption of different organic substances such as the drugs to be released or eluted. For the coating of the stent with the drug, the bare metal stent is contacted with a solution or dispersion, which contains the drug to be released. The coating solution or dispersion fills the micropores completely and creates a uniform layer after evaporation of the solvent.

Bare metal stents are commercially available, e.g. from Boston Scientific, Guidant, Medtronic, Biotronik, Abbot, Cordis, Johnson & Johnson.

A drug-eluting stent system with a dose-adjustable, polymer-free, on-site stent coating has already been used in the prevention of restenosis (Mehilli et al., European Heart Journal 2005; 26; 1475-1481).

Besides paclitaxel, sirolimus and tacrolimus, various anti-inflammatory and antiproliferative substances such as everolimus, ABT-578, biolimus, QP2, dexamethasone, 17-β-estradiol, batimastat, actinomycin-D, methotextrat, angiopeptin, tyrosinkinase inhibitors, vincrystin, mitomycin, cyclosporin, and also the C-myc antisense technology (resten-NG,AVI-4126) have been tested as drugs in drug-eluting stents (Silber, Z Kardiol 93:649-663 (2004)).

It is further known that endothelin, a peptide hormone, which is a potent vasoconstrictor, mitogen and stimulant of collagen synthesis may play a central role in forming restenosis after coronary stenting.

Endothelin-1 has been implicated in the pathogenesis of restenosis since circulating levels are elevated after PTCA (Katwa et al., Basic Res Cardiol 1999, 94; 445-453): It promotes neointima formation after ballon angioplasty in the rat (Douglas et al., J Cardiovasc Pharmacol 1993, 8; 371 - 373).

Conversely, an active endothelin receptor antagonist suppresses neointimal development in balloon-injured rat arteries (Ferrer et al., J Cardiovasc Pharmacol 1995, 26; 908-915), in pigs (Burke et al., J Cardiovasc Pharmacol1997, 30:33-41) and in coronary arteries of pigs (Dashwood et al., Cardiovasc Res 1999, 43:445-456; McKenna et al., Circulation 1998, 97:2551-2556; Kirchengast, J Cardiovasc Pharmacol 2001, 38: 31-34).

First results in human tissue showed that endothelin receptor antagonists reduced intimal hyperplasia in an organ culture of human saphenous vein grafts (Porter et al., J Vasc Surg 1998, 28:697-701).

In a double blind study using bosentan, a receptor antagonist of endothelin, in a cohort of patients with coronary artery disease, the coronary diameter increased. Bosentan IV could safely be given and was well tolerated (Wenzel et al., Circulation 1998, 98: 2235-2240). In human, IMA endothelin receptor blockade showed beneficial effects on endothelin-dependent vasorelaxation (Verma et al., Cardiovasc Res 2001; 1:146-152).

However, although the parenteral administration of endothelin antagonists such as tezosentan and bosentan to patients suffering from coronary heart insufficiency ameliorated the condition, the data concerning morbidity and mortality were disappointing. Neither for mortality nor for the combined endpoint of mortality and hospitalism due to coronary heart insufficiency a significant advantage for bosentan could be found. To the contrary, the hospitalism rate tended to be even higher (Kindermann und Böhm, http://www.cardiovasc.de/hefte/2002/Sonderheft 1/40.htm).

Starting form the referenced prior art, the problem to be solved was to provide an improved alternative for the treatment of conditions associated with stenosis. This alternative should provide an improved efficacy in the treatment of the addressed conditions and/or should reduce adverse effects as far as possible.

This problem was solved by a polymer-free stent, which comprises an endothelin receptor antagonist.

In a preferred embodiment, the stent is a bare metal stent.

The term "stent" includes medical devices or implants, with or without polymer coating on the surface, which are implanted in the lumen of the vessel, e.g. via a catheter, or on the exterior of the vessel, e.g. as a mesh or covering. The stent may also become a part of the vessel itself, for example to replace a portion of a diseased or traumatized vessel, e.g. as a synthetic graft.

The term "bare metal" means that the stent is made of metal, wherein no polymeric layer is applied on the surface which could serve as adsorber of the endothelin receptor antagonist to be eluted/released from said. The endothelin receptor antagonist to be released is directly absorbed on the surface of the metal, the stent is made of.

Accordingly, the term "polymer-free" defines the complete absence of polymers within the stent. The term "polymer" relates to substances composed of repeating structural units or monomers, connected with covalent chemical bonds. The term "polymer" does not include macrocyclic compounds that have an effect as endothelin receptor antagonist.

The term "comprising" includes terms such as "coated" or "embedded". The term "coated" with reference to a stent is meant to be interpreted broadly to include compounds on the surface or otherwise integral to the bare metal stent such that the therapeutic compound elutes or is released from the surface of the bare metal stent after implantation in the subject.

The term "endothelin receptor antagonist" encompasses all respective antagonists known from the prior art. It is possible that the polymer-free stent comprises one or more endothelin receptor antagonists.

The term "endothelin receptor antagonist" also includes the pharmaceutically acceptable derivatives thereof. A "pharmaceutically-acceptable derivative" denotes any salt, conjugate, isomers, ester, prodrug, derivative, or a metabolite or residue thereof.

The endothelin receptor antagonists can either be peptides or non-peptides.

Non-peptide endothelin receptor antagonists are preferred.

Non-peptide endothelin receptor antagonists are for example tezosentan, bosentan, sitaxsentan, atrasentan, LU 127043, LU 135252, LU 224332, LU 302872.

These compounds are known compounds and are commercially available.

Tezosentan is N-{6-(2-hydroxyethoxy)-5-(2-methoxyphenoxy)-2-[2-(1H-tetrazol-5-yl)-4-pyridyl]-pyrimidin-4-yl}-5-isopropylpyridin-2-sulfonamide sometimes also termed as [5-isopropyl-pyridine-2-sulphonic acid 6-(2-hydroxy-ethoxy)-5-(2-methoxy-phenoxy)-2-(2-1H-tetrazol-5-yl-pyridin-4-yl)-pyrimidin-4-ylamide]. It is registered in the Chemical Abstracts under the CAS no 180384-57-0. It is sold under the trademark Veletri®. Tezosentan is an endothelin receptor antagonist with a high affinity to both endothelin A (ET_{A} ) and B (ET_{B}) receptors (dual action).

Bosentan is N-[6-(2-hydroxyethoxy)-5-(2-methoxyphenoxy)-2-pyrimidin-2-yl-pyrimidin-4-yl]-4-tert-butyl-benzenesulfonamide or 4-tert-butyl-N-[6-(2-hydroxyethoxy)-5-(2-methoxyphenoxy)-2-(pyrimidin-2-yl)pyrimidin-4-yl]-benzenesulfonamide (CAS no 147536-97-8. It is sold under the trademark Tracleer®.

Sitaxsentan is N-(4-chloro-3-methyl-oxazol-5-yl)-2-[2-(6-methylbenzo[1,2]dioxol-5-yl)acetyl]thiophene-3-sulfonamide (CAS no 210421-64-0). It is sold under the trademark Thelin®.

Atrasentan is (2R,3R,4S)-1-[dibutylcarbamoyl)methyl]-2-(p-methoxyphenyl)-4-[3,4-(methylendioxy)phenyl]-3-pyrrolidincarboxylic acid (CAS no 173937-91-2). It is sold under the trademark Xinlay®.

LU 127043 is (2-[(4,6-dimethoxy-pyrimidin-2-yl)oxy]-3-methoxy-3,3-diphenyl-propionic acid and LU 135252 is the (+)S enantiomer thereof (Riechers et al., J med Chem 39:2123-2128 (1996)).

LU 224332 is [2-(4,6-dimethyl-pyrimidin-2-yl)oxy]-3-[2-(3,4-dimethoxyphenyl)ethoxy]-3,3-diphenyl-propionic acid and LU 302872 is the (+)S enantiomer thereof (Raschak et al., J Cardiovasc Pharmacol 31 (Suppl. 3), 241-244 (1998)).

In a preferred embodiment, said endothelin receptor antagonist is selected from the group consisting of tezosentan, bosentan, sitaxsentan, atrasentan, LU 127043, LU 135252, LU 224332, LU 302872.

In a more preferred embodiment, said endothelin receptor antagonist is selected from the group consisting of tezosentan, bosentan, sitaxsentan.

The most preferred endothelin receptor antagonist is tezosentan. The endothelin receptor antagonist on the surface of the stent can also be present in the form of a pharmaceutical composition.

Said pharmaceutical composition comprises a therapeutically effective amount of an endothelin receptor antagonist, effective to treat and/or prevent and/or ameliorate restenosis or stenosis or in-stent-restenosis of a mammalian vessel traumatized by a surgical procedure, and a pharmaceutically acceptable carrier. Preferably, the pharmaceutical acceptable carrier is, but not limited to, a liposome, nanoparticle, microparticle, emulsion, gel, paste or mixed micelles. Some examples of materials which can serve as pharmaceutically acceptable carriers are, but not limited to, sugars such as lactose, glucose and sucrose, starches such as corn starch, hydroxyethyl starch, cellulose and its derivatives such as sodium carboxymethyl cellulose, ethyl cellulose and cellulose acetate, hydroxypropylmethyl cellulose, tragacanth; malt; gelatine, talc; excipients such as cocoa butter and suppository waxes; oils such as peanut oil; cottonseed oil; safflower oil, sesame oil, olive oil, corn oil and soybean oil; glycols, such as propylene glycol; esters such as ethyl oleate and ethyl laureate; agar; buffering agents such as magnesium hydroxide and aluminium hydroxide; alginic acid; pyrogen-free water; isotonic saline; Ringer's solution; ethyl alcohol, and phosphate buffer solutions, as well as other non-toxic compatible lubricants such as sodium lauryl sulphate and magnesium stearate, as well as releasing agents, coating agents, preservatives and antioxidants.

Pharmaceutical compositions of the endothelin receptor antagonist are further, but not limited to, dispersions, suspensions (nanosuspensions, microsuspensions), emulsions (nanoemulsions, microemulsions, self-emulsifying systems), solutions, liposomes, microparticles, nanoparticles, mixed micells and hydrosols and other forms of colloid disperse systems known to a person skilled in the art. Further, materials that are biodegradable and non-toxic, bio-adhesive or mucoadhesive excipients such as hyaluronic acid or chitosan or stearates, cross-linked derivates of polyacrylic acid, polyvinyl alcohol, polyvinylpyrrolidone, polysaccharides and saponin, poly(lactic acid), poly(glycolic acid), poly(lactide-co-glycolide), polycaprolactone (PCL), chitosan, alginates, poly(ethylene oxide) (PEO), poly(2-hydroxyethyl methacrylate) (PHEMA), polyethylene glycol (PEG), polyvinylpyrollidone (PVP) or any combination thereof, are acceptable pharmaceutical carriers.

These compositions may also contain preservative agents, wetting agents, emulsifying agents, and dispersing agents. Prevention of the action of microorganisms may be ensured by various antibacterial and antifungal agents, for example, parabens, chlorobutanol, phenol, sorbic acid, and the like. It may also be desirable to include isotonic agents, for example, sugars, sodium chloride and the like.

The pharmaceutical composition containing the endothelin receptor antagonist adopts the appropriate release or eluting properties or eluting properties. These release properties are tailored through the appropriate pharmaceutical dosage forms known to the person skilled in the art. These release properties are, but not limited to, sustained release, extended release, modified release or any other form of controlled release.

"Release" or "eluting" means a dosage form designed to release a therapeutic agent for a period from about 0.0005 to about 180, preferably from about 1 to 150, and more preferably from about 30 about 120 days. Release or eluting over a longer period of time is also contemplated as release in the context of the present invention.

The term "about" as used herein is intended to reflect a variation of 20 % of the value it is attached to.

The term "active pharmaceutical ingredient" refers to all structures, which are chemically active and all known chemical forms thereof. Examples are, but not limited to, conjugates, isomers, esters, derivatives, metabolites, residues, salts or prodrugs thereof.

"Therapeutically effective amount" refers to an amount of a compound of the present invention alone or an amount of the combination of compounds claimed or an amount of a compound of the present invention in combination with other active ingredients effective to treat the diseases described herein.

The composition and the stent provided herein is preferably sterile and is preferably non-pyrogenic, e.g. containing less than 1 EU (Endotoxin Unit, a standard measure) and preferably less than 0.1 EU per dose or application.

In one embodiment, the stent has an individual dose adjustment. Dose adjustment may be desirable to enable an individual dose adjustment for specific lesion or patient subsets, e.g. higher doses or lower doses for patients with special conditions such a diabetes or other chronic disease. Further, the present invention pertains to a stent with multiple compounds, i.e. the endothelin receptor antagonists and for instance compounds which inhibit smooth muscle cell proliferation and promote re-endothelialization, anti-cloting or reduce high cholesterol levels.

In another embodiment, the present invention relates to a polymer-free stent comprising multiple coatings of the endothelin receptor antagonists or endothelin receptor antagonists and further pharmacological active compounds. Such pharmacological active compounds are but not limited to thrombolytics (e.g. reteplase, tenecteplase, anistreplase, streptokinase, urokinase), anticoagulants (e.g. heparin, warfarin, acenocoumarol, phenprocoumon, phenindione, argatroban, lepirudin, bivalirudin), antibiotics, corticosteroids (e.g. beclomethasone, budesonide, betamethasone, ciclesonide, flunisolide, fluticasone, mometasone, refleponide, triamcinolone), antiplatelet agents (e.g. aspirin, clopidogrel, ticlopidine, abciximab, eptifibatide, tirofiban, cilostazol), non-stereoidal anti-inflammatories (NSAID) (e.g. salicylates (e.g. aspirin, amoxiprin, benorilate, choline magnesium salicylate, diflunisal, faislamine, methyl salicylate, magnesium salicylate, salicyl salicylate), arylalkanoic acids (e.g. diclofenac, aceclofenac, acemetacin, bromfenac, etodolac, indometacin, nabumetone, sulindac, tometin), 2-Arylpropionic acids (profens) (e.g. ibuprofen, carprofen, fenbufen, fenoporfen, flurbiprofen, ketoprofen, ketorolac, loxoprofen, naproxen, oxaprozin, tiaprofenic acid, suprofen), N-Arylanthranilic acids (fenamic acids) (e.g. mefenamic acid, meclofenamic acid), pyrazolidine derivates (e.g. phenylbutazone, azapropazone, metamizole, oxyphenbutazone, sulfinpyrazone), oxicams (e.g. piroxicam, lornoxicam, meloxicam, tenoxicam), COX-2 inhibitors (e.g. celecoxib, etoricoxib, lumiracoxib, parecoxib, rofecoxib, valdecoxib), sulphonanilides (e.g. nimesulide)), growth factors (e.g. transforming growth factor beta (TGF-β), granulocyte-colony stimulating factor (G-CSF), granulocyte-macrophage colony stimulating factor (GM-CSF), nerve growth factor (NGF), neurotrophins, platelet derived growth factor (PDGF), erythropoietin (EPO), thrombopoietin (TPO), myostatin (GDF-8), growth differentiation factor-9 (GDF9), acidic fibroblast growth factor (aFGF or FGF-1), basic fibroblast growth factor (bFGF or FGF-2), epidermal growth factor (EGF), hepatocyte growth factor (HGF)), immunosuppressant drugs (e.g. cyclosporine, tacrolimus, prednisolone, azathioprine, sirolimus, mycopehnolate, glatiramer acetate), antineoplastic agents (alkylating agents (e.g. cisplatin, carboplatin, oxaliplatin, mechlorethamine, cyclophosphamide, chlorambucil), anti-metabolites (e.g. azathioprine, mercaptopurine, pyrimidine), vinca alkaloids (e.g. vincristine, vinblastine, vinorelbine, vindesine), podophyllotoxin (e.g. etoposide, teniposide), taxanes (e.g. paclitaxel), topoisomerase inhibitors (e.g. amsacrine, etoposide, etoposide phosphate, teniposide, camptothecins such as irinotecan, topotecan), antitumor antibiotics (e.g. dactinomycin), monoclonal antibodies (e.g. trastuzumab, cetuximab, rituximab, bevacizumab)), ACE-inhibitors (e.g. captopril, zofenopril, enalapril, ramipril, quinapril, perindopril, lisinopril, benazepril, fosinopril), HMG-coA reductase inhibitors (e.g. atorvastatin, cerivastatin, fluvastatin, lovastatin, pravastatin, simvastatin), beta-blockers (e.g. alprenolol, carteolol, levobunolol, mepindolol, metipranolol, nadolol, oxprenolol, penbutolol, pindolol, propranolol, sotalol, timolol, acebutolol, atenolol, betaxolol, bisoprolol, esmolol, metoprolol, nebivolol, carvedilol, celiprolol, lavetalol, butaxamin), calcium channel blockers (e.g. verapamil, gallopamil, diltiazem, nimodipine, amlodipine, felodipine, nicardipine, nifedipine, nisoldipine, nitrendipine, lacidipine, lercandipine), long acting nitrates (e.g. isosorbide dinitrate and mononitrate, nitroglycerin) and cholesterol-lowering agents (e.g. bezafibrate, ciprofibrate, clofibrate, gemfibrozil, fenofibrate).

The stent is for use as medicament.

Specifically, the stent is for the treatment of stenosis.

The term "treatment" also encompasses term such as "preventing" or "ameliorating" a certain condition associated with stenosis.

The term "stenosis" is defined to be a narrowing in a blood vessel or other tubular organ or structure, i.e. a vasoconstricting condition. This term also encompasses terms such as "restenosis" and "in-stent restenosis". The term "restenosis" refers to the re-occurence of stenosis. Restenosis may occur, in the arteries or other blood vessels and/or any hollow organ. The term "in-stent restenosis (ISR)" refers to an inflammatory reaction centred at the stent struts, the severity of which is related to the trauma to the vessel wall induced by the stent struts.

The term "blood vessel" includes, but is not limited to, coronary, femoral, carotid and peripheral vessels of a mammal.

The term "proliferation" as used herein means an increase of the cell number, i.e. by mitosis of the cells.

In another embodiment, the stent is for the treatment of coronary artery disease.

The term "coronary artery disease" means all conditions associated with vasoconstricting conditions or stenosis that occurs in the coronary artery.

The term "peripheral obliterative arterial disease (POAD)" means all conditions associated with peripheral arteries and/or vessels. The terms peripheral vascular disease (PVD), peripheral arterial disease (PAD), peripheral obliterative arterial disease (POAD) and peripheral artery occlusive disease (PAOD) are used within the scope of this invention as equivalent terminologies.

Accordingly, the stent is for the treatment of peripheral arterial disease (PAD).

The manufacture of the polymer-free stent is performed by dissolving or dispersing the endothelin receptor antagonist in a suitable solvent. Suitable solvents are preferably solvents which can easily be removed from the coated stent, e.g. by evaporation. Such a solvent is e.g. ethanol. The polymer containing or the polymer-free, i.e. the bare metal stent, is contacted with the prepared solution or dispersion in order to allow the dispersion or solution to enter the micropores of the stent material. After a time sufficient for the solution or dispersion to fill the pores of the polymer-free stent, the stent is removed from the solution or dispersion. The solvent is removed by evaporation, thereby producing the polymer-free stent comprising the endothelin receptor antagonist.

Accordingly, the process for the manufacture of the stent is **characterized in that** it comprises step (i):
(i) contacting a stent with an endothelin receptor antagonist.

The methods for the implantation of the stent to a traumatized mammalian vessel leading to or involving intimal proliferation are state of the art. For example, the stent can be applied via a catheter.

Traumatization may be associated with or caused by medical procedures. Said medical procedures are, but not limited to, an angioplasty procedure, coronary arteriography, a coronary artery bypass graft (CABG) operation, atherectomy, rotablation, laser, ultrasound.

The implantation of the stent is either carried out during the procedure which traumatizes the vessel and/or before and/or after the trauma appears. That means that the stent may be used for prevention and/or treatment and/or amelioration of the vascular trauma of the vessel.

The invention also provides a kit comprising, separately packaged, an implantable stent adapted for the delivery of the endothelin receptor antagonist and a unit dosage form comprising the amount of the endothelin receptor antagonist for the treatment of stenosis or for the treatment of a coronary artery disease. The unit dosage form is, but not limited to, an ethanolic solution, an emulsion, suspension, nanoparticles, microparticles, mixed micelles or liposomes of the endothelin receptor antagonist. Preferably, the kit also comprises a second unit dosage form comprising an additional active pharmaceutical ingredient. The endothelin receptor antagonist delivered within the kit can be used alone or in combination with the other active pharmaceutical ingredients, e.g. the ingredients mentioned above. The delivery of the dispersed microparticles, nanoparticles, liposomes, ethanolic solution of the active pharmaceutical ingredient to said traumatized mammalian vessel is effective to inhibit or reduce diminution in vessel lumen, diameter in the vessel.

Accordingly, the invention encompasses a kit comprising an implantable stent and an endothelin receptor antagonist. By contacting said stent and said endothelin receptor antagonist, the stent comprising the endothelin receptor antagonist is obtained.

The invention is now described with reference to the following examples. These examples are provided for the purpose of illustration of the invention only and the invention should not be construed as being limited to these examples, but rather should be construed to encompass any and all variations which become evident as a result of the teaching provided herein. The following materials and methods are provided with respect to the subsequent examples but do not limit a multiplicity of materials and methodologies encompassed by the present invention.

### EXAMPLES

### Example 1: Coating of a bare metal stent with tezosentan

Tezosentan was dispersed in ethanol (96%, pharmaceutical quality). The resulting emulsion was stirred until a clear solution of tezosentan in ethanol was obtained. The end concentration of tezosentan in ethanol was 2 mg/ml. Bare metal stents were coated with the 2 mg/ml tezosentan-ethanol solution by employing the Translumina Stent Coating Machine (T-SCM 2003). Simple coatings (1 x 2 mg/ml) yielded very thin film, whereas multiple coatings (3 x 2 mg/ml) yielded a thicker film. The said thick film, due to multiple coating, was represented by a visible white layer. The tezosentan amount on the stent coating was detected by employing UV-visible spectroscopy at 276 nm. After coating, the stent was diluted by washing the stent with 2 ml of pharmaceutical ethanol for a minute. Three stent systems Yukon^{®} 3008 were employed. After three times coating with 2 mg/ml tezosentan-ethanol solution about 50 µg - 150 µg of tezosentan was detected on the stents - depending on the stent length. After dilution, about 25 µg - 75 µg of tezosentan was left over on each stent, e.g. 73,08 µg of tezosentan was detected on a 24 mm stent.

The quantification measurements demonstrated that one yielded about 25 µg of tezosentan on a 8 mm Translumina Yukon^{®} stent by employing a 2 mg/ml tezosentan-ethanol coating solution after three times coating.

The same experimental procedure was repeated by employing a 20 mg/ml tezosentan-ethanol coating solution. After running the coating procedure only once about 123 µg of tezosentan was yielded on the stent.

## Claims

1. Polymer-free stent comprising an endothelin receptor antagonist.

2. Stent of claim 1 **characterized in that** it is a bare metal stent.

3. Stent of claim 1 or 2 , **characterized in that** said endothelin receptor antagonist is selected from the group consisting of tezosentan, bosentan, sitaxsentan, atrasentan, LU 127043, LU 135252, LU 224332, LU 302872.

4. Stent of any one of the preceding claims, **characterized in that** said endothelin receptor antagonist is selected from the group consisting of tezosentan, bosentan, sitaxsentan.

5. Stent of any one of the preceding claims , **characterized in that** said endothelin receptor antagonist is tezosentan.

6. Stent of any one of the preceding claims , **characterized in that** it elutes said endothelin receptor antagonist.

7. Stent of any one of the preceding claims for use as a medicament.

8. Stent of any one of the preceding claims for the treatment of stenosis.

9. Stent of any one of the preceding claims for the treatment of coronary artery diseases.

10. Stent of any one of the preceding claims for the treatment of peripheral arterial disease.

11. Stent of any one of the preceding claims **characterised in that** it comprises at least one active pharmaceutical ingredient selected from the group of thrombolytics, anticoagulants, antibiotics, corticosteroids, antiplatelet agents, non-stereoidal anti-inflammatories (NSAID), arylalkanoic acids, 2-Arylpropionic acids (profens), N-Arylanthranilic acids (fenamic acids), pyrazolidine derivates, oxicams, COX-2 inhibitors, sulphonanilides, growth factors (e.g. transforming growth factor beta (TGF-β), granulocyte-colony stimulating factor (G-CSF), granulocyte-macrophage colony stimulating factor (GM-CSF), nerve growth factor (NGF), neurotrophins, platelet derived growth factor (PDGF), erythropoietin (EPO), thrombopoietin (TPO), myostatin (GDF-8), growth differentiation factor-9 (GDF9), acidic fibroblast growth factor (aFGF or FGF-1), basic fibroblast growth factor (bFGF or FGF-2), epidermal growth factor (EGF), hepatocyte growth factor (HGF)), immunosuppressant drugs, antineoplastic agents (alkylating agents, anti-metabolites, vinca alkaloids, podophyllotoxin, taxanes, topoisomerase inhibitors, antitumor antibiotics, monoclonal antibodies), ACE-inhibitors, HMG-coA reductase inhibitors, beta-blockers, calcium channel blockers, long acting nitrates and cholesterol-lowering agents or any combination thereof.

12. Process for the manufacture of a stent as defined in any one of claims 1 to 11, **characterized in that** it comprises step (i):
(i) contacting a stent with an endothelin receptor antagonist.

13. Kit comprising an implantable stent and an endothelin receptor antagonist, wherein by contacting said stent with said endothelin receptor antagonist the stent as defined in any one of claims 1 to 11 is obtained.
